# DEMANDE DE BREVET EUROPEEN

(11) **EP 0 551 035 A1**
(43) Date de publication de la demande: **14.07.1993**
(21) Numéro de dépôt: 92403434.1
(22) Date de dépôt: 17.12.1992
(51) Int. Cl.: C07C 69/743, A01N 53/00, C07F 7/08, C07D 207/333, C07D 277/24, C07C 255/39, C07C 61/40, C07C 61/35

(54) **Nouveaux dérivés de l'acide 2,2-diméthyl cyclopropane carboxylique portant en 3 une chaîne but-1-èn-3-ynyle, leur procédé de préparation et leur application comme pesticides**

(30) Priorité: 20.12.1991 FR 9115857; 21.02.1992 FR 9202009
(71) Demandeur: ROUSSEL UCLAF, F-93230 Romainville (FR)
(72) Inventeur: Benoit, Marc, F-13360 Roquevaire (FR); Demoute, Jean-Pierre, F-93360 Neuilly Plaisance (FR); Girodeau, Jean-Marc, F-77500 Chelles (FR)
(74) Mandataire: Vieillefosse, Jean-Claude

(57) **Abrégé**

L'invention à pour objet les composés de formule (I) : 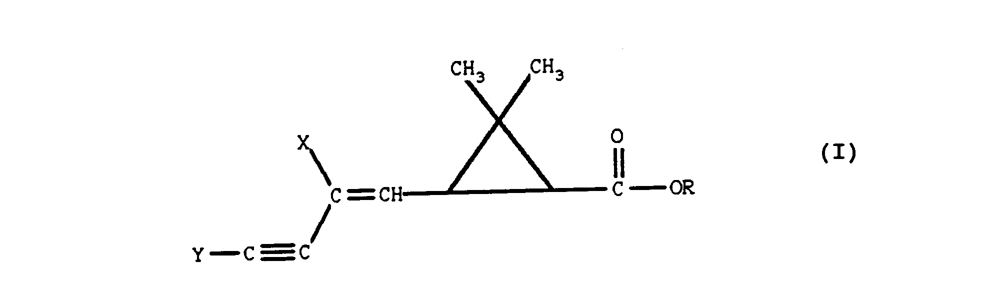
dans lesquels :
- X représente un atome d'hydrogène, un atome d'halogène, un radical alkyle, éventuellement substitué,
- Y représente un atome d'hydrogène, un atome d'halogène, un radical alkyle, éventuellement substitué, un radical aryle ou aralkyle, éventuellement substitué, un radical (CH₂)ₘ Si(alc₁)₃, un radical (CH₂)ₙ Oalc₂ ou (CH₂)ₚ Salc₃, m, n et p représentant un nombre entier pouvant varier de 0 à 6, alc₁, alc₂ et alc₃ représentant un radical alkyle et R représente un radical alkyle comprenant de 1 à 18 atomes de carbone ou le reste d'un alcool utilisé en série pyréthrinoïde.

Les composés de formule (I) sont doués de propriétés pesticides.

## Description

La présente invention concerne de nouveaux dérivés de l'acide 2,2-diméthyl cyclopropanecarboxylique portant en 3 une chaîne but-1-èn-3-ynyle, leur procédé de préparation et leur application comme pesticides.

Les composés de formule (I) : 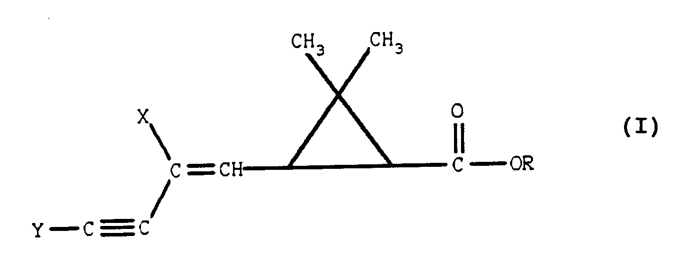
sous toutes leurs formes stéréoisomères possibles ainsi que leurs mélanges dans lesquels :
- X représente un atome d'hydrogène, un atome d'halogène, un radical alkyle renfermant jusqu'à 8 atomes de carbone, linéaire, ramifié ou cyclique, saturé ou insaturé, éventuellement substitué par un ou plusieurs atomes d'halogène,
- Y représente un atome d'hydrogène, un atome d'halogène, un radical alkyle linéaire, ramifié ou cyclique, saturé ou insaturé, renfermant jusqu'à 8 atomes de carbone, éventuellement substitué par un ou plusieurs atomes d'halogène, par un ou plusieurs radicaux CN, par un ou plusieurs radicaux hydroxyle ou alkyloxy, ou Y représente un radical aryle ou aralkyle renfermant jusqu'à 16 atomes de carbone éventuellement substitué par un ou plusieurs atomes d'halogène, par un ou plusieurs radicaux alkyle ou alkoxy renfermant jusqu'à 8 atomes de carbone, ou Y représente un radical (CH₂)ₘ Si(alc₁)₃, un radical (CH₂)ₙ Oalc₂ ou (CH₂)ₚ Salc₃, m, n et p représentant un nombre entier pouvant varier de 0 à 6, alc₁, alc₂ et alc₃ représentant un radical alkyle linéaire, ramifié ou cyclique renfermant jusqu'à 8 atomes de carbone et R représente un radical alkyle renfermant jusqu'à 18 atomes de carbone ou le reste d'un alcool utilisé en série pyréthrinoïde.

Lorsque X ou Y représente un atome d'halogène, il s'agit de préférence d'un atome de fluor, de chlore ou de brome.

Lorsque X ou Y représente un radical alkyle, il s'agit de préférence d'un radical méthyle, éthyle, n-propyle, isopropyle, n-butyle, isobutyle, tert-butyle, pentyle, hexyle, vinyle, 1,1-diméthylallyle, éthynyle, propynyle, cyclopropyle, cyclobutyle, cyclopentyle ou cyclohexyle, éventuellement substitué par un ou plusieurs atomes de fluor, de chlore ou de brome, comme valeur de X on peut citer notamment le radical CF₃.

Aryle représente de préférence un radical phényle.

Aralkyle représente de préférence un radical benzyle ou phénéthyle.

alc₁, alc₂ et alc₃ représentent de préférence un radical méthyle, éthyle ou propyle.

Lorsque Y représente un radical aryle ou aralkyle substitué par un ou plusieurs radicaux alkyle ou alkoxy, on entend de préférence par radical alkyle, un radical méthyle, éthyle, isopropyle, n-propyle, n-butyle, isobutyle ou tert-butyle, et par radical alkoxy un radical méthoxy, éthoxy, n-propoxy, isopropoxy, n-butoxy, isobutoxy ou tert-butoxy.

Lorsque Y représente un radical alkyle, aryle ou aralkyle substitué par un ou plusieurs atomes d'halogène, l'halogène concerné est de préférence un atome de fluor, de chlore ou de brome.

Parmi les composés préférés de l'invention, on peut citer les composés dans lesquels X représente un atome d'hydrogène, de fluor ou un radical trifluorométhyle, ceux dans lesquels Y représente un atome d'hydrogène, ceux dans lesquels Y représente un radical alkyle renfermant jusqu'à 6 atomes de carbone, par exemple un radical méthyle, isopropyle ou tert-butyle, ceux dans lesquels Y représente un radical Si(alc₁)₃ dans lequel alc₁ conserve sa signification précédente, par exemple un radical méthyle.

Parmi les composés préférés de l'invention, on peut citer les composés dans lesquels la géométrie de la double liaison est cis.

L'invention a plus particulièrement pour objet les composés de formule (I), dans lesquels R représente :
- soit un radical alkyle renfermant de 1 à 18 atomes de carbone,
- soit un radical benzyle éventuellement substitué par un ou plusieurs radicaux choisis dans le groupe constitué par :
   . les atomes d'halogène et notamment le fluor,
   . les radicaux alkyle, alkényle ou alkynyle, O-alkyle, O-alkényle ou O-alkynyle, S-alkyle, S-alkényle ou S-alkynyle, linéaires, ramifiés ou cycliques renfermant jusqu'à 8 atomes de carbone éventuellement substitués par un ou plusieurs atomes d'halogène et notamment par un ou plusieurs atomes de fluor,
   . les radicaux NO₂, CN, NH₂,
- soit un radical : 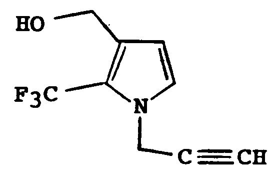
- soit un groupement : 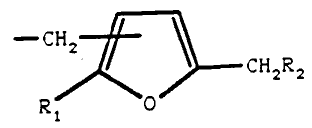 dans lequel le substituant R₁ représente un atome d'hydrogène ou un radical méthyle et le substituant R₂ un aryle monocyclique ou un groupement -CH₂-C≡CH et notamment un groupement (5-benzyl 3-furyl) méthyle,
- soit un groupement : 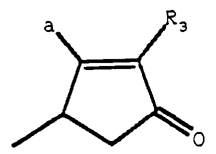 dans lequel a représente un atome d'hydrogène ou un radical méthyle et R₃ représente un radical organique aliphatique comportant de 2 à 6 atomes de carbones et une ou plusieurs insaturations carbone-carbone et notamment le radical -CH₂-CH=CH₂, -CH₂-CH=CH-CH₃, -CH₂-CH=CH-CH=CH₂, -CH₂-CH=CH-CH₂-CH₃,
- soit un groupement : 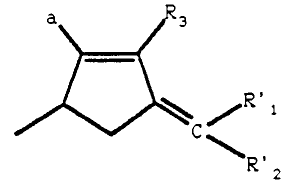 dans lequel a représente un atome d'hydrogène ou un radical méthyle, R₃ conserve la même signification que précédemment, R'₁ et R'₂, identiques ou différents, représentent un atome d'hydrogène, un atome d'halogène, un radical alkyle renfermant de 1 à 6 atomes de carbone, un radical aryle comportant de 6 à 10 atomes de carbone, un groupement alkyloxycarbonyle comportant de 2 à 5 atomes de carbone, ou un grougement cyano,
- soit un groupement : 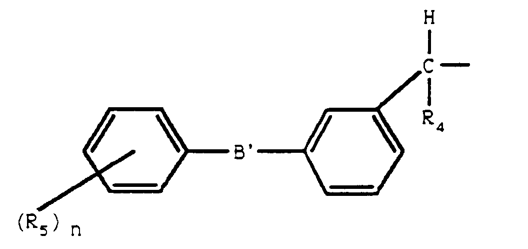 dans lequel B' représente un atome d'oxygène ou de soufre ou un groupement 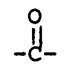 ou -CH₂- ou un groupement sulfoxyde ou un groupement sulfone et R₄ représente un atome d'hydrogène, un radical -C≡N, un radical méthyle, un radical -CONH₂, un radical -CSNH₂ ou un radical -C≡CH, R₅ représente un atome d'halogène ou un radical méthyle et n représente un nombre égal à 0, 1 ou 2, et notamment le groupement 3-phénoxy benzyle, alpha-cyano 3-phénoxy benzyle, alpha-éthynyl 3-phénoxy benzyle, 3-benzoyl benzyle, 1-(3-phénoxy phényl) éthyle ou alpha-thioamido 3-phénoxy benzyle,
- soit un groupement : 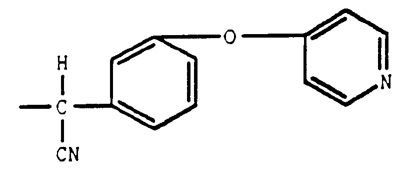
- soit un groupement : 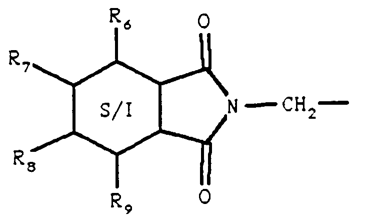 dans lequel les substituants R₆, R₇, R₈, R₉ représentent un atome d'hydrogène, un atome de chlore, ou un radical méthyle et dans lequel S/I symbolise un cycle aromatique ou un cycle analogue dihydro ou tétrahydro,
- soit un groupement : 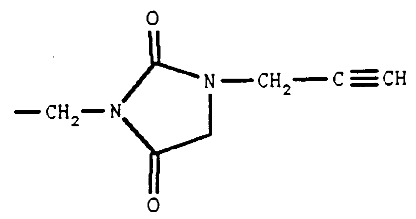
- soit un groupement : 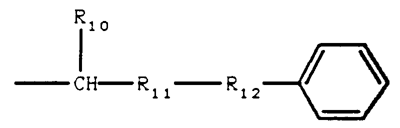 dans lequel R₁₀ représente un atome d'hydrogène ou un radical CN, R₁₂ représente un radical -CH₂- ou un atome d'oxygène, R₁₁ représente un radical thiazolyle ou thiadiazolyle dont la liaison avec 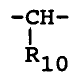 peut se trouver à l'une quelconque des positions disponibles, R₁₂ étant lié à R₁₁ par l'atome de carbone compris entre l'atome de soufre et un atome d'azote,
- soit un groupement : 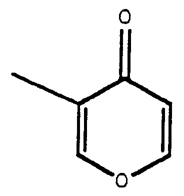
- soit un groupement : 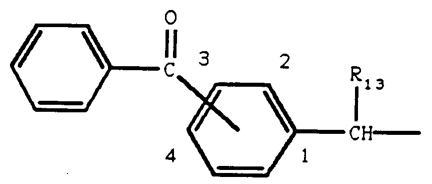 dans lequel R₁₃ représente un atome d'hydrogène, un radical méthyle, éthynyle ou cyano et le radical benzoyle est en position 3 ou 4,
- soit un groupement : 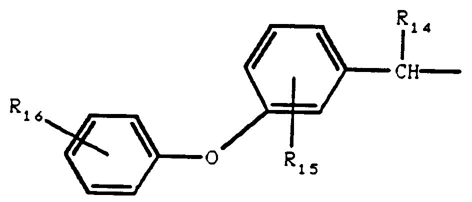 dans lequel R₁₄ a la signification indiquée ci-dessus pour R₁₃ et, R₁₅ et R₁₆, différents, représentent un atome d'hydrogène, de fluor ou de brome,
- soit un groupement : 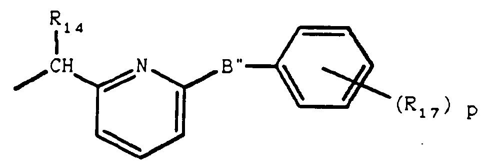 dans lequel R₁₄ est défini comme ci-dessus, chacun des R₁₇ représente indépendamment un groupement alkyle renfermant de 1 à 4 atomes de carbone, alkoxy renfermant de 1 à 4 atomes de carbone, alkylthio renfermant de 1 à 4 atomes de carbone, alkylsulfonyle renfermant de 1 à 4 atomes de carbone, trifluorométhyle, 3,4-méthylène dioxy, chloro, fluoro ou bromo, p représente un nombre égal à 0, 1 ou 2 et B" représente un atome d'oxygène ou un atome de soufre,
- soit un groupement 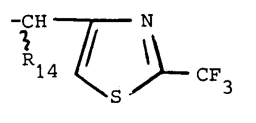 dans lequel R₁₄ a la signification indiquée ci-dessus.

L'invention a tout spécialement pour objet les composés de formule (I) dans lesquels R représente un radical : 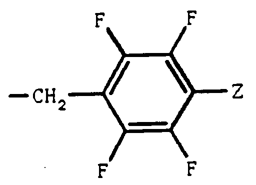
dans lesquels Z représente un atome d'hydrogène, un atome de fluor, un radical NH₂, un radical alkyle, alkényle ou alkynyle, O-alkyle, O-alkynyle, S-alkyle et S-alkényle, renfermant jusqu'à 8 atomes de carbone, linéaire ramifié ou cyclique, éventuellement substitué par un ou plusieurs atomes d'halogène et notamment par un ou plusieurs atomes de fluor. Parmi les valeurs préférées de Z on peut citer H, F, CH₃, CF₃, OCF₃, OCHF₂, CH₂C≡CH et CH₂-CH=CH₂.

Parmi les composés préférés de l'invention, on peut citer, les composés de formule (I) dans lesquels R représente un radical :

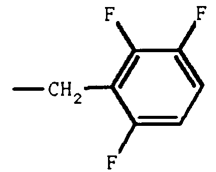

L'invention a naturellement pour objet les composés dont la préparation est donnée ci-après dans la partie expérimentale et notamment les produits des exemples 1, 14, 22, 29, 30, 35, 39, 40 et 41, ou encore ceux des exemples 54 et 55. Parmi les composés de l'invention, on peut citer tout spécialement les composés des exemples 33, 35, 43 et 45.

L'invention a également pour objet un procédé de préparation, caractérisé en ce que l'on soumet un acide de formule (II) : 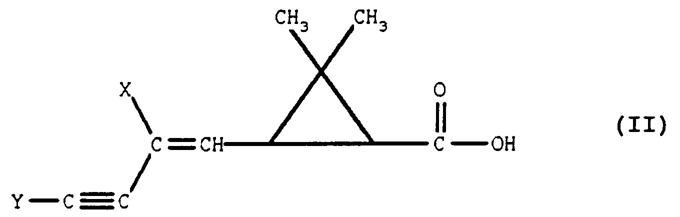
dans laquelle X et Y conservent leur signification précédente, ou un dérivé fonctionnel de cet acide à l'action d'un alcool de formule (III) :

ROH (III)

dans laquelle R conserve sa signification précédente ou d'un dérivé fonctionnel de cet alcool pour obtenir le composé de formule (I) correspondant.

L'estérification de l'acide de formule (II) avec l'alcool de formule (III) peut être effectuée en présence d'une base tertiaire telle que la pyridine. Cette estérification peut être effectuée avantageusement en présence d'un mélange de pyridine, de dicyclohexyl carbodiimide et de 4-diméthyl amino pyridine.

L'estérification peut également être réalisée en faisant réagir un chlorure de l'acide (II) sur l'alcool de formule (III) ou sur un dérivé métallique de cet alcool tel qu'un sel d'argent.

Les composés de formule (II) utilisés comme produits de départ du procédé de l'invention sont des produits nouveaux et sont en eux-mêmes un objet de la présente invention.

Des exemples détaillés de préparation des composés de formule (II) sont donnés ci-après dans la partie expérimentale, leur préparation peut être schématisée comme suit : 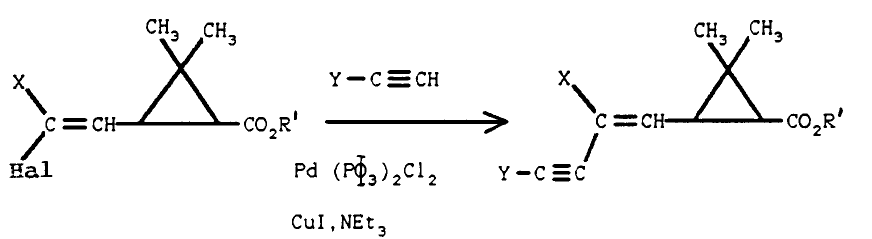
R' représentant un radical alkyle comportant de 1 à 18 atomes de carbone, ou un reste d'un acide utilisé en série pyréthrinoïde et Hal représentant un atome de brome, de chlore ou d'iode 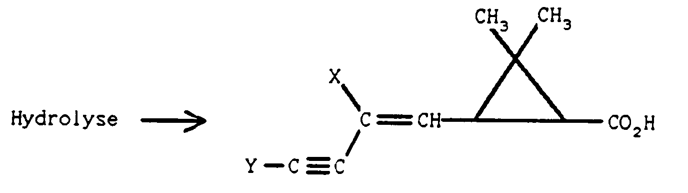
Si on le souhaite on sépare les stéréoisomères obtenus.

L'invention a également pour objet une variante du procédé précédent, caractérisé en ce que l'on soumet un composé de formule (IV) : 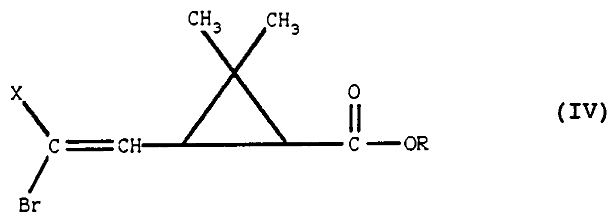
à l'action d'un agent susceptible de remplacer l'atome de brome par le radical C≡CY pour obtenir le composé de formule (I) correspondant.

Les composés de formule (I) présentent d'intéressantes propriétés qui permettent leur utilisation dans la lutte contre les parasites. Il peut s'agir par exemple de la lutte contre les parasites des végétaux, qu'il s'agisse des parasites du sol ou des parties aériennes, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent aussi être utilisés pour lutter contre les insectes et autres parasites du sol, par exemple les coléoptères, comme DIABROTICA, les taupins et les vers blancs, les myriapodes comme les scutigérelles et les blaniules, et les diptères comme les cécydomies et les lépidoptères comme les noctuelles terricoles.

Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) sont de plus photostables et sont peu toxiques pour les mammifères.

L'ensemble de ces propriétés fait des produits de formule (I) des produits qui correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques du genre de Boophilus, ceux du genre Hyalomnia, ceux du genre Amblyomnia et ceux du genre Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule (I) définis ci-dessus, notamment les produits des exemples 1, 14, 22, 29, 30, 39, 40 et 41 ou encore ceux des exemples 54 et 55 et tout particulièrement ceux des exemples 33, 35, 43 et 45.

Parmi les produits de formule (I) définis ci-dessus, on peut citer plus spécialement comme produits destinés à la lutte contre Diabrotica, les produits contre Blatella, les produits des exemples 22, 32, 34, 48, 49, 50, 51 et 52, comme produits destinés à la lutte contre les Lepidoptères, les produits des exeples 46 et 47 et comme produits destinés à la lutte contre Aphis, on peut citer plus spécialement les produits des exemples 1, 47 et 53.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet les compositions acaricides renfermant comme principe actif au moins un des produits de formule I définie ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2,2-1]hept-5-ène-2,3-dicarboximide, ou le pipéronyl-bis-2-(2'-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcool alpha-cyano 3-phénoxy benzylique des acides 2,2-diméthyl 3-(2,2-dichlorovinyl) cyclopropane-1-carboxyliques, par les esters d'alcool alpha-cyano 3-phénoxy benzylique d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les exemples suivants illustrent l'invention sans toutefois la limiter.

### EXEMPLE 1 : [1R-[1alpha, 3alpha(Z)]] 2,2-diméthyl 3-[4-(triméthylsilyl) but-1-èn-3-ynyl] cyclopropanecarboxylate de 2,3,5,6-tétrafluoro 4-(2-propynyl) phénylméthyle

On ajoute à 0 +5°C une solution de 1,75 g de dicyclohexylcarbodiimide et 3 cm³ de chlorure de méthylène dans une solution renfermant 2,12 g d'acide [1R-[1alpha, 3alpha(Z)]] 2,2-diméthyl 3-[4-(triméthylsilyl) but-1-èn-3-ynyl] cyclopropane carboxylique, préparé comme indiqué ci-après, 20 cm³ de chlorure de méthylène, 2 g d'alcool 2,3,5,6-tétrafluoro 4-(2-propynyl) benzylique et 20 mg de diméthylamino pyridine. On maintient le mélange réactionnel sous agitation pendant 1 heure à 0 +5°C, puis pendant 2 heures à 20°C. On filtre, rince et concentre le filtrat. On obtient 4,53 g de produit que l'on chromatographie sur silice en éluant avec le mélange hexane-chlorure de méthylène (70-30). On obtient 1,34 g du produit recherché rf = 0,23.
[alpha]_{D} = 117°5 (c = 1 % CHCl₃).

### PREPARATION 1 : Acide [1R-[1alpha, 3alpha(Z)]] 2,2-diméthyl 3-[4-(triméthylsilyl) but-1-èn-3-ynyl] cyclopropanecarboxylique

On ajoute à 15°C, 3,47 g de chlorotriméthylsilane dans un mélange de 6,75 g d'acide [1R-[1alpha, 3alpha(Z)]] 3-[2-bromo éthényl] 2,2-diméthyl cyclopropanecarboxylique, obtenu selon EP 0381563, 70 cm³ de chlorure de méthylène et 2,31 g d'imidazole. On maintient le mélange réactionnel sous agitation pendant 1 h 30 à 20°C. On filtre et amène à sec. On obtient 7,37 g de produit auquel on ajoute 110 cm³ de triéthylamine anhydre et 7 cm³ de triméthylsilylacétylène. On maintient le mélange réactionnel sous agitation en ajoutant à 20°C, 0,91 g de dichlorure de bis(triphénylphosphine) palladium et 0,16 g d'iodure de cuivre. On maintient le mélange réactionnel sous agitation pendant 5 heures à 45°C, puis 16 heures à 20°C. On filtre et amène à sec. On ajoute 100 cm³ d'une solution aqueuse saturée en phosphate acide de sodium. On extrait à l'éther isopropylique. On réunit les phases organiques. On sèche, filtre et amène à sec. On obtient 9,4 g de produit que l'on chromatographie sur silice, éluant toluène-acétate d'éthyle-acide acétique (95-5-0,1). On isole ainsi le produit recherché. rf = 0,16.

### EXEMPLE 2 : [1R-[1alpha, 3alpha(E)]] 2,2-diméthyl 3-[4-(triméthylsilyl) but-1-èn-3-ynyl] cyclopropanecarboxylate de (pentafluorophényl) méthyle,

En opérant comme à l'exemple 1, à partir de l'acide [1R-[1alpha, 3alpha(E)]] 2,2-diméthyl 3-[4-(triméthylsilyl) but-1-èn-3-ynyl] cyclopropanecarboxylique préparé comme indiqué ci-après et de l'alcool pentafluorobenzylique, on obtient le produit recherché fondant à 91°C.

### PREPARATION 2: acide [1R-[1alpha, 3alpha(E)]] 2,2-diméthyl 3-[4-(triméthylsilyl) but-1-èn-3-ynyl] cyclopropanecarboxylique

En opérant comme à la préparation 1, à partir de l'acide [1R-[1alpha, 3alpha(E)]] 3-(2-bromo éthényl) 2,2-diméthyl cyclopropanecarboxylique obtenu selon EP 0381563, on obtient le produit recherché.

### EXEMPLES 3 à 13 :

En opérant comme à l'exemple 1, à partir de l'acide [1R-[1alpha, 3alpha(Z)]] 2,2-diméthyl 3-[4-(triméthylsilyl) but-1-èn-3-ynyl] cyclopropanecarboxylique (préparation 1) ou de l'acide [1R-[1alpha, 3alpha(E)]] 2,2-diméthyl 3-[2-fluoro 4-(triméthylsilyl) but-1-èn-3-ynyl] cyclopropanecarboxylique (préparation 3) et de l'alcool approprié, on a préparé les produits suivants :

### EXEMPLE 3 : [1R-[1alpha, 3alpha(Z)]] 2,2-diméthyl 3-[4-(triméthylsilyl) but-1-èn-3-ynyl] cyclopropanecarboxylate de [2-fluoro 6-(trifluorométhyl) phényl] méthyle,

F = 39°5.

### EXEMPLE 4 : [1R-[1alpha, 3alpha(Z)]] 2,2-diméthyl 3-[4-(triméthylsilyl) but-1-èn-3-ynyl] cyclopropanecarboxylate de [(4-difluorométhoxy) 2,4,5,6-tétrafluoro phényl] méthyle,

rf = 0,13, éluant hexane-éther isopropylique (97-3).

### EXEMPLE 5 : [1R-[1alpha, 3alpha(Z)]] 2,2-diméthyl 3-[4-(triméthylsilyl) but-1-èn-3-ynyl] cyclopropanecarboxylate de [1-(2-propynyl) 2-(trifluorométhyl) 1-pyrrol 3-yl] méthyle,

[alpha]_{D} = 139° (c = 1,1 % CHCl₃).

### EXEMPLE 6 : [1R-[1alpha, 3alpha(Z)]] 2,2-diméthyl 3-[4-(triméthylsilyl) but-1-èn-3-ynyl] cyclopropanecarboxylate de [(4-amino 2,3,5,6-tétrafluoro) phényl] méthyle,

rf = 0,2, éluant hexane-acétate d'éthyle (85-15).

### EXEMPLE 7 : [1R-[1alpha, 3alpha(Z)]] 2,2-diméthyl 3-[4-(triméthylsilyl) (but-1-èn-3-ynyl) cyclopropanecarboxylate de (1,3,4,5,6,7-hexahydro-1,3-dioxo-2H-isoindol-2-yl) méthyle,

F = 87,9°.

### EXEMPLE 8 : [1R-[1alpha, 3alpha(Z)]] 2,2-diméthyl 3-[4-(triméthylsilyl) but-1-èn-3-ynyl] cyclopropanecarboxylate de S (3-phénoxy phényl) cyanométhyle

F = 82,2°.

### EXEMPLE 9 : [1R-[1alpha, 3alpha(Z)]] 2,2-diméthyl 3-[4-(triméthylsilyl) but-1-èn-3-ynyl] cyclopropanecarboxylate de (2,3,5,6-tétrafluoro 4-méthyl phényl) méthyle,

rf = 0,23, éluant hexane-chlorure de méthylène (70-30).

### EXEMPLE 10 : [1R-[1alpha, 3alpha(Z)]] 2,2-diméthyl 3-[4-(triméthylsilyl) but-1-èn-3-ynyl] cyclopropanecarboxylate de (2,3,5,6-tétrafluoro phényl) méthyle,

[alpha]_{D} = + 137,5° (C = 1 % CHCl₃).

### EXEMPLE 11 : [1R-[1alpha, 3alpha(E)]] 2,2-diméthyl 3-[2-fluoro 4-(triméthylsilyl) but-1-èn-3-ynyl] cyclopropanecarboxylate de (2,3,5,6-tétrafluoro phényl) méthyle,

rf ≈ 0,13 éluant hexane 80 CH₂Cl₂20

### EXEMPLE 12 : [1R-[1alpha, 3alpha(E)]] 2,2-diméthyl 3-[2-fluoro 4-(triméthylsilyl) but-1-èn-3-ynyl] cyclopropanecarboxylate de (2,6-difluoro phényl) méthyle,

rf = 0,18 éluant hexane 97 éther iso 3

### EXEMPLE 13 : [1R-[1alpha, 3alpha(E)]] 2,2-diméthyl 3-[2-fluoro 4-(triméthylsilyl) but-1-èn-3-ynyl] cyclopropanecarboxylate de (2,3,6-trifluoro phényl) méthyle,

rf = 0,16 éluant hexane 80 CH₂Cl₂20

### PREPARATION 3 : Acide [1R-[1alpha, 3alpha(E)]] 2,2-diméthyl 3-[2-fluoro 4-(triméthylsilyl) but-1-èn-3-ynyl] cyclopropanecarboxylique,

En opérant comme à la préparation 1, à partir de 14,6 g d'acide [1R-[1alpha, 3alpha(Z)]] 3-[2-bromo 2-fluoro éthényl] 2,2-diméthyl cyclopropanecarboxylique obtenu selon EP 0378026, on obtient 10,56 g de produit recherché. rf = 0,10 éluant hexane 90 ACOEt 10 ACOH1.

### PREPARATION 4 : Alcool 2,3,6-trifluoro benzylique

On dissout à la température ambiante, sous atmosphère d'azote 10,56 g d'acide 2,3,6-trifluoro benzoïque dans 100 cm³ de tétrahydrofuranne. On refroidit au bain de glace-méthanol et ajoute en 30 minutes une solution renfermant 10 cm³ de complexe borane méthyl sulfure à 10 millimoles/cm³ et 30 cm³ de tétrahydrofuranne. On agite encore pendant 5 minutes et laisse revenir à la température ambiante. On chauffe pendant 3 h 30 à 45/50°C. On verse la solution obtenue sur une solution aqueuse de phosphate acide de sodium. On extrait à l'éther isopropylique, lave, sèche et amène à sec. On obtient 10,6 g de liquide incolore que l'on chromatographie sur silice en éluant avec le mélange hexane-acétate d'éthyle (7-3). On obtient 8,8 g de produit recherché. F < 50°C. Spectre IR (CHCl₃)
- OH: 3620 cm⁻¹
- Aromatique: 1642, 1604, 1499 cm⁻¹

Spectre de RMN (CDCl₃, 60 Hz) ppm
- Protons aromatiques: 6,70 - 7,47
- CH₂-OH: 4,83
- OH: 2,20

### EXEMPLE 14 : [1R-[1alpha, 3alpha(Z)]] 3-(2-fluoro 5,5-diméthyl hex-1-èn-3-ynyl) 2,2-diméthyl cyclopropanecarboxylate de (2,3,5,6-tétrafluoro 4-méthyl phényl) méthyle et son isomère (E) correspondant.

On maintient sous agitation pendant 20 heures à 45/50°C, un mélange de 2 g de [1R-[1alpha, 3alpha(E+Z)]] 3-(2-bromo 2-fluoro éthényl) 2,2-diméthyl cyclopropanecarboxylate de 4-méthyl tétrafluorobenzyle obtenu selon EP 0381563, 0,720 cm³ de 3,3-diméthyl 1-butyne, 20 cm³ de triéthylamine, 170 mg de bis(triphénylphosphine) palladium dichlorure et 26 mg d'iodure de cuivre. On verse le milieu réactionnel sur un mélange d'eau et de glace. On extrait à l'acétate d'éthyle. On sèche et concentre à sec. On chromatographie sur silice le produit obtenu en éluant avec le mélange hexane-chlorure de méthylène (7-3). On obtient 1,13 g de produit recherché, isomère E, F = 47°C (rf = 0,20)
[alpha]_{D} = +108,5° (C = 1,1 % CHCl₃)
et 0,7 g d'isomère Z : rf = 0,27
[alpha]_{D} = -77° (C = 0,6 % CHCl₃)

### EXEMPLES 15 à 21 :

En opérant comme à l'exemple précédent, en suivant le schéma réactionnel : 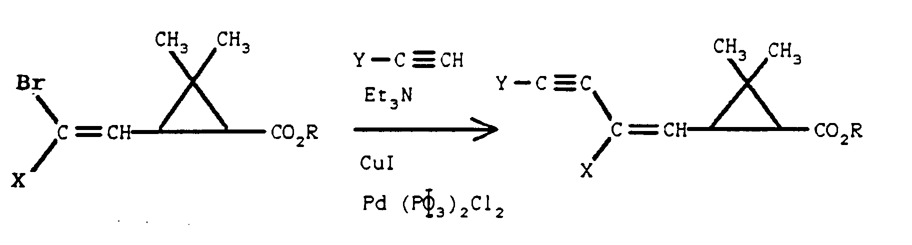
on a obtenu les produits suivants :

### EXEMPLE 15 : [1R-[1alpha, 3alpha(Z)]] 3-(2-fluoro pent-1-èn-3-ynyl) 2,2-diméthyl cyclopropanecarboxylate de (2,3,5,6-tétrafluoro 4-méthyl phényl) méthyle

F = 84°C et isomère E correspondant (Rf 0,24 Hexane 7-CH₂Cl₂ 3)

### EXEMPLE 16 : [1R-[1alpha, 3alpha(E)]] 3-(2-fluoro hex-1-èn-3-ynyl) 2,2-diméthyl cyclopropanecarboxylate de (2,3,5,6-tétrafluoro 4-méthyl phényl) méthyle

Rf 0,28 (Hexane 7-chlorure de méthylène 3) et isomère Z correspondant.

### EXEMPLE 17 : [1R-[1alpha, 3alpha(E)]] 3-(2-fluoro 4-phényl but-1-èn-3-ynyl) 2,2-diméthyl cyclopropanecarboxylate de (2,3,5,6-tétrafluoro 4-méthyl phényl) méthyle

[alpha]_{D} = +191°5 (0,75 % CHCl₃)

### EXEMPLE 18 : [1R-[1alpha, 3alpha(E)]] 3-(2-fluoro 4-triméthylsilyl but-1-èn-3-ynyl) 2,2-diméthyl cyclopropanecarboxylate de (pentafluorophényl) méthyle

En utilisant au départ le [1R-[1alpha, 3alpha(E+Z)]] 3-(2-bromo 2-fluoroéthényl) 2,2-diméthyl cyclopropanecarboxylate de pentafluorobenzyle obtenu selon EP 0381563, on a obtenu le produit attendu.
[alpha]_{D} = +106° (2,2 % CHCl₃) et l'isomère Z correspondant [alpha]_{D} = -89° (1,1 % CHCl₃)

### EXEMPLE 19 : [1R-[1alpha, 3alpha(Z)]] 3-[2-fluoro 4-(triméthylsilyl) but-1-èn-3-ynyl] 2,2-diméthyl cyclopropanecarboxylate de (2,3,5,6-tétrafluoro 4-méthyl phényl) méthyle

F < 50°C et isomère E correspondant.

### EXEMPLE 20 : [1R-[1alpha, 3alpha(Z)]] 3-[2-fluoro 5-(triméthylsilyl) pent-1-èn-3-ynyl] 2,2-diméthyl cyclopropanecarboxylate de (2,3,5,6-tétrafluoro 4-méthyl phényl) méthyle,

[alpha]_{D} = -93,5° (0,7 % CHCl₃) et isomère (E) correspondant [alpha]_{D} = +140° (0,7 % CHCl₃)

### EXEMPLE 21 : [1R-[1alpha, 3alpha(Z)]] 2,2-diméthyl 3-(4-phényl but-1-èn-3-ynyl) cyclopropanecarboxylate de (2,3,5,6-tétrafluoro 4-méthyl phényl) méthyle,

En utilisant au départ le [1R-[1alpha, 3alpha(Z)]] 3-(2-bromoéthényl) 2,2-diméthyl cyclopropanecarboxylate de 4-méthyl tétrafluorobenzyle obtenu selon EP 0381563, on a obtenu le produit attendu.
[alpha]_{D} = + 199° (C = 1 % CHCl₃).

### EXEMPLE 22 : [1R-[1alpha, 3alpha(Z)]] 3-(but-1-èn-3-ynyl) 2,2-diméthyl cyclopropanecarboxylate de [2,3,5,6-tétrafluoro 4-(2-propynyl) phényl] méthyle

On ajoute à 0° + 5°C, 1,37 g de fluorure de potassium dans une solution renfermant 2,04 g de produit de l'exemple 1, 20 cm³ de méthanol et 0,47 cm³ d'acide acétique. On agite 5 minutes à 0° + 5°C puis 16 heures à 20°C. On concentre, reprend à l'eau et extrait à l'éther isopropylique. On sèche, filtre et amène à sec. On obtient 1,85 g d'un produit que l'on chromatographie sur silice en éluant avec le mélange hexane-éther isopropylique (97-3). On obtient 0,913 g du produit recherché. F = 61°C.
En opérant comme à l'exemple 22, en suivant le schéma réactionnel

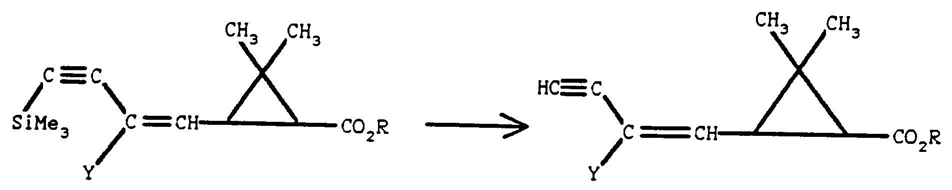

On a obtenu les produits suivants :

### EXEMPLE 23 : [1R-[1alpha, 3alpha(Z)]] 3-(but-1-èn-3-ynyl) 2,2-diméthyl cyclopropanecarboxylate de [2-fluoro 6-(trifluorométhyl) phényl] méthyle

En utilisant 1,83 g du produit obtenu à l'exemple 3, on a obtenu 1,19 g du produit recherché.
rf = 0,5
Eluant hexane-éther isopropylique (95-5).

### EXEMPLE 24 : [1R-[1alpha, 3alpha(Z)]] 3-(but-1-èn-3-ynyl) 2,2-diméthyl cyclopropanecarboxylate de [2,3,5,6-tétrafluoro 4-(difluorométhoxy) phényl] méthyle

A partir de 1,43 g du produit obtenu à l'exemple 4, on a recueilli 1,02 g du produit recherché.
rf = 0,2
Eluant hexane-éther isopropylique (95-5).

### EXEMPLE 25 : [1R-[1alpha, 3alpha(Z)]] 3-(but-1-èn-3-ynyl) 2,2-diméthyl cyclopropanecarboxylate de [1-(2-propynyl) 2-(trifluorométhyl) 1-pyrrol-3-yl] méthyle

A partir de 1,1 g du produit obtenu à l'exemple 5, on a obtenu 0,83 g du produit recherché.
rf = 0,13
Eluant hexane-éther isopropylique (97-3).

### EXEMPLE 26 : [1R-[1alpha, 3alpha(E)]] 3-(but-1-èn-3-ynyl) 2,2-diméthyl cyclopropanecarboxylate de (pentafluorophényl) méthyle

A partir de 2,28 g du produit obtenu à l'exemple 2, on a obtenu 1,4 g du produit recherché.
[alpha]_{D} = -90° (C = 1 % CHCl₃).

### EXEMPLE 27 : [1R-[1alpha, 3alpha(Z)]] 3-(2-fluoro but-1-èn-3-ynyl) 2,2-diméthyl cyclopropanecarboxylate de (2,3,5,6-tétrafluoro 4-méthyl phényl) méthyle

A partir de 1,5 g du produit recueilli à l'exemple 19 (isomère Z), on a préparé 1,24 g du produit recherché
(F = 58°C).

### EXEMPLE 28 : [1R-[1alpha, 3alpha(Z)]] 3-(but-1-èn-3-ynyl) 2,2-diméthyl cyclopropanecarboxylate de (2,3,5,6-tétrafluoro 4-méthyl phényl) méthyle

A partir de 1,5 g de produit de l'exemple 9, on a obtenu 1,1 g du produit recherché.
rf = 0,2 éluant hexane-éther isopropylique (97-3).

### EXEMPLE 29 : [1R-[1alpha, 3alpha(E)]] 3-(2-fluoro but-1-èn-3-ynyl) 2,2-diméthyl cyclopropanecarboxylate de (pentafluorophényl) méthyle

A partir de 1,24 g de produit de l'exemple 18 (isomère E) on a obtenu 990 mg du produit recherché.
[alpha]_{D} = +89° (C = 1,6 % CHCl₃).

### EXEMPLE 30 : [1R-[1alpha, 3alpha(E)]] 3-(2-fluoro but-1-èn-3-ynyl) 2,2-diméthyl cyclopropanecarboxylate de (2,3,5,6-tétrafluoro 4-méthyl phényl) méthyle

A partir de 4,1 g de produit de l'exemple 19 (isomère E), on a obtenu 3,36 g du produit recherché. F < 50°C.
rf = 0,23, éluant hexane-chlorure de méthylène (7-3).

### EXEMPLE 31 : [1R-[1alpha, 3alpha(Z)]] 3-(2-fluoro but-1-èn-3-ynyl) 2,2-diméthyl cyclopropanecarboxylate de (pentafluorophényl) méthyle

A partir de 1 g du produit de l'exemple 18 (isomère Z), on a obtenu 840 mg de produit recherché. F ≃ 57°C.
[alpha]_{D} = -66° (C = 1 % CHCl₃)

### EXEMPLE 32 : [1R-[1alpha, 3alpha(Z)]] 3-(but-1-èn-3-ynyl) 2,2-diméthyl cyclopropanecarboxylate de (4-amino 2,3,5,6-tétrafluoro phényl) méthyle

A partir de 2,11 g de composé de l'exemple 6, on a obtenu 1,4 g du produit recherché.
F = 80,2°C.

En opérant à partir des composés obtenus aux exemples 11 à 13 selon le mode opératoire indiqué à l'exemple 22, on a obtenu respectivement les produits des exemples 33 à 35.

### EXEMPLE 33 : [1R-[1alpha, 3alpha(E)]] 2,2-diméthyl 3-(2-fluoro but-1-èn-3-ynyl) cyclopropanecarboxylate de (2,3,5,6-tétrafluoro phényl) méthyle

rf = 0,18 cyclohexane-chlorure de méthylène 7-3.

### EXEMPLE 34 : [1R-[1alpha, 3alpha(E)]] 2,2-diméthyl 3-(2-fluoro but-1-èn-3-ynyl) cyclopropanecarboxylate de (2,6-difluoro phényl) méthyle

rf = 0,18 cyclohexane-chlorure de méthylène 7-3.

### EXEMPLE 35 : [1R-[1alpha, 3alpha(E)]] 2,2-diméthyl 3-(2-fluoro but-1-èn-3-ynyl) cyclopropanecarboxylate de (2,3,6-trifluoro phényl) méthyle

rf ≃ 0,2 éluant hexane 70 CH₂Cl₂ 30.

### EXEMPLE 36 : [1R-[1alpha, 3alpha(Z)]] 3-(but-1-èn-3-ynyl) 2,2-diméthyl cyclopropanecarboxylate de (2,3,5,6-tétrafluoro phényl) méthyle

On ajoute à 0°C, 60 cm³ de fluorure de tétrabutylammonium, en solution molaire dans le tétrahydrofuranne, dans une solution renfermant 2,44 g de produit préparé à l'exemple 10 et 30 cm³ de chlorure de méthylène. On maintient le mélange réactionnel sous agitation pendant 30 minutes à 0°C et laisse la température remonter à 15°C. On verse le milieu réactionnel sur une solution aqueuse saturée en phosphate acide de sodium et agite pendant 10 minutes. On extrait à l'éther isopropylique. On réunit les phases organiques, sèche, filtre et amène à sec. On obtient 1,96 g d'un produit que l'on chromatographie sur silice en éluant avec le mélange hexane-éther isopropylique (98-2). On isole ainsi 1,20 g de produit recherché. (rf = 0,16).
[alpha]_{D} = +105,5° (C = 0,75 % CHCl₃).

### EXEMPLE 37 : [1R-[1alpha, 3alpha(Z)]] 2,2-diméthyl 3-[4-(triméthylsilyl) but-1-èn-3-ynyl] cyclopropanecarboxylate de (pentafluorophényl) méthyle

Le produit a été obtenu selon le mode opératoire de l'exemple 14 en utilisant au départ le [1R-[1alpha, 3alpha]] 2,2-diméthyl 3-(2-bromoéthényl) cyclopropane carboxylate de 2,3,4,5,6-pentafluorophényl méthyle obtenu selon EP 0381563.
[alpha]_{D} = + 113,5° (C = 0,45 % CHCl₃).

### EXEMPLE 38 : [1R-[1alpha, 3alpha(Z)]] 3-(but-1-èn-3-ynyl) 2,2-diméthyl cyclopropanecarboxylate de (pentafluorophényl) méthyle

Le produit a été obtenu à partir de l'exemple 37 en suivant le mode opératoire de l'exemple 36.
[alpha]_{D} = + 129° (C = 0,95 % CHCl₃).

### EXEMPLE 39 : [1R-[1alpha, 3alpha(E)]] 3-(2-fluoro 5,5-diméthyl hex-1-èn-3-ynyl) 2,2-diméthyl cyclopropanecarboxylate de (2,3,5,6-tétrafluoro phényl) méthyle

Le produit a été obtenu selon le mode opératoire de l'exemple 14.
Rf 0,12 (hexane 8-CH₂Cl₂ 2).

### EXEMPLE 40 : [1R-[1alpha, 3alpha(E)]] 3-(2-fluoro 5,5-diméthyl hex-1-èn-3-ynyl) 2,2-diméthyl cyclopropanecarboxylate de (pentafluorophényl) méthyle

Le produit a été obtenu selon le mode opératoire de l'exemple 14.
Rf 0,16 (hexane 8-CH₂Cl₂ 2).

### EXEMPLE 41 : [1R-[1alpha, 3alpha(E)]] 3-(2-fluoro 5-méthyl hex-1-èn-3-ynyl) 2,2-diméthyl cyclopropanecarboxylate de [(2,3,5,6-tétrafluoro 4-méthyl) phényl] méthyle

Rf 0,22 (hexane 8-CH₂Cl₂ 2).

### EXEMPLE 42 : [1R-[1alpha, 3alpha(E)]] 3-(2-trifluorométhyl) 4-(triméthylsilyl) but-1-èn-3-ynyl) 2,2-diméthyl cyclopropanecarboxylate de (2,3,6-trifluorophényl) méthyle

En opérant comme à l'exemple 1 à partir de l'acide [1R-[1alpha, 3alpha (E)]] 2,2-diméthyl 3-[2-(trifluorométhyl) 4-(triméthylsilyl) but-1-èn-3-ynyl] cyclopropane carboxylique et de l'alcool approprié, on obtient le produit attendu.

### PREPARATION 5 : Acide [1R-[1alpha, 3alpha (E)]] 2,2-diméthyl 3-[2-(trifluorométhyl) 4-(triméthylsilyl) but-1-èn-3-ynyl] cyclopropane carboxylique

En opérant comme à la préparation 1 en utilisant au départ l'acide [1R-[1alpha, 3alpha (E)]] 3-[2-bromo 2-(trifluorométhyl)] éthényl cyclopropane carboxylique obtenu selon le brevet européen 010874 ou le brevet français 2 392 964, on obtient le produit recherché.

### EXEMPLE 43 : [1R-[1alpha, 3alpha(E)]] 3-(2-trifluorométhyl) but-1-èn-3-ynyl) 2,2-diméthyl cyclopropane carboxylate de (2,3,6-trifluorophényl) méthyle.

En opérant comme à l'exemple 22 à partir du produit obtenu à l'exemple 42, on obtient le produit attendu.
F = 48°C.

### EXEMPLE 44 : [1R-[1alpha, 3alpha(E)]] 3-(2-trifluorométhyl) 4-(triméthylsilyl-but-1-èn-3-ynyl) 2,2-diméthyl cyclopropane carboxylate de (2,3,5,6-tétrafluorophényl) méthyle.

En opérant comme à l'exemple 42 à partir de l'acide obtenu à la préparation 5 et de l'alcool approprié, on obtient le produit attendu.

### EXEMPLE 45 : [1R-[1alpha, 3alpha(E)]] 3-(2-trifluorométhyl) but-1-èn-3-ynyl) 2,2-diméthyl cyclopropane carboxylate de (2,3,5,6-tétrafluorophényl) méthyle.

En opérant comme à l'exemple 43 à partir du produit obtenu à l'exemple 44, on obtient le produit attendu.
F = 63,2°C.

### EXEMPLE 46 : [1R-[1alpha(S*), 3alpha(Z)]] 3-(2-fluoropent-1-èn-3-ynyl) 2,2-diméthyl cyclopropane carboxylate de cyano (3-phénoxyphényl) méthyle.

En opérant comme à l'exemple 1 à partir de l'acide [1R-[(1alpha, 3alpha (Z)]] 2,2-diméthyl 3-[2-fluoroéthényl pent-1**-**èn-3-ynyl] cyclopropane carboxylique et de l'alcool approprié, on obtient le produit attendu.

### PREPARATION 6 : Acide [1R-[(1alpha, 3alpha (Z)]] 2,2-diméthyl 3-[2-fluoroéthényl pent-1-èn-3-ynyl] cyclopropane carboxylique

On refroidit à +10°C, 7,3 g d'acide [1R-[(1alpha, 3alpha (Z)] 2,2-diméthyl 3-(2-bromo 2-fluoroéthényl) cyclopropane carboxylique obtenu selon EP 0378026 dans 70 cm³ de chlorure de méthylène, ajoute 2,09 g d'imidazole puis 4,3 cm³ de chloro triméthylsilane. On agite en laissant revenir à température ambiante, filtre, évapore le filtrat et obtient 9,25 g d'ester silylé. On refroidit à -5°C, 20 cm³ d'une solution de méthyl acétylène à 14% dans le triéthylamine, ajoute 50 cm³ de triéthylamine, 70 cm³ d'acétonitrile, 9,25 g d'ester silylé obtenu ci-dessus, 210 mg de dichlorure de bis (triphénylphosphine) palladium et 3( mg d'iodure de cuivre et maintient 4 heures à 60°C. On verse sur une solution aqueuse saturée en phosphate de sodium, extrait à l'acétate d'éthyle, sèche et évapore à sec. On obtient 4,1 g de produit attendu.

En opérant comme indiqué dans les exemples précédents en utilisant au départ l'acide et l'alcool appropriés, on obtient les produits suivants.

### EXEMPLE 47 : [1R-[1alpha(S*), 3alpha(E)]] 3-(2-fluoro 4-triméthylsilyl but-1-èn-3-ynyl) 2,2-diméthyl cyclopropane carboxylate de cyano (3-phénoxyphényl) méthyle.

F = 103,3°C.

### EXEMPLE 48 : [1R-[1alpha, 3alpha(E)]] 3-(2-fluoro but-1-èn-3-ynyl) 2,2-diméthyl cyclopropane carboxylate de [4-(2-propynyl) 2,3,5,6-tétrafluorophényl) méthyle.

Rf ≈ 0,13.

### EXEMPLE 49 : [1R-[1alpha, 3alpha(Z)]] 3-(5,5-diméthyl hex-1-èn-3-ynyl) 2,2-diméthyl cyclopropane carboxylate de 2,3,5,6-tétrafluorophényl) méthyle.

Rf ≈ 0,15 (hexane-AcOEt 98-2).

### PREPARATION 7 : Acide [1R-[1alpha, 3alpha(Z)]] 2,2-diméthyl 3-(5,5-diméthyl hex-1-èn-3-ynyl) cyclopropane carboxylique.

On opère comme à la préparation 1 à partir de l'acide [1R-[(1alpha, 3alpha (Z)] 2,2-diméthyl 3-[2-(bromoéthényl)] cyclopropane carboxylique obtenu selon EP 0381563 et du 1-(triméthylsilyl) 1,1-diméthyl 2-butyne et obtient l'acide attendu.

### EXEMPLE 50 : [1R-[1alpha, 3alpha(Z)]] 3-(2-fluoro but-1-èn-3-ynyl) 2,2-diméthyl cyclopropane carboxylate (4-hydroxyméthyl 2,3,5,6-tétrafluorophényl) méthyle.

F = 74°C. Rf = 0,09 (éluant CH₂Cl₂)

### EXEMPLE 51 : [1R-[1alpha(S*), 3alpha(E)]] 3-(2-trifluorométhyl but-1-èn-3-ynyl) 2,2-diméthyl cyclopropane carboxylate de cyano (3-phénoxyphényl) méthyle.

Rf ≈ 0,25 (CH₂Cl₂-hexane 50-50).

### EXEMPLE 52 : [1R-[1alpha, 3alpha(E)]] 3-(2-trifluorométhyl) but-1-èn-3-ynyl) 2,2-diméthyl cyclopropane carboxylate de [1-(2-trifluorométhyl) 4-thiazolyl] 2-propynyle.

Rf ≈ 0,2 (hexane-CH₂Cl₂ 7-3).

### PREPARATION 8 : alpha-éthynyl 2-(trifluorométhyl) 4-thiazolyl méthanol.

On ajoute 11 cm³ d'une solution molaire de bromure d'éthynyl magnésium dans une solution de tétrahydrofuranne renfermant 2 g de 2-(trifluorométhyl) 4-thiazolecarboxaldéhyde. On maintient le mélange réactionnel sous agitation pendant 30 minutes à 20 ≃ 25°C. On verse sur une solution de chlorure d'ammonium. On extrait au chlorure de méthylène, sèche, filtre et amène à sec. On obtient 2,1 g de produit recherché.

### EXEMPLE 53 : [1R-[1alpha, 3alpha(E)]] 3-(2-fluoro 4-triméthylsilyl but-1-èn-3-ynyl) 2,2-diméthyl cyclopropane carboxylate de [4-(2-propynyl) 2,3,5,6-tétrafluorophényl)] méthyle.

Rf ≈ 0,15 (hexane-CH₂Cl₂ 7-3).

### EXEMPLE 54 : [1R-[1alpha, 3alpha(E)]] 3-(2-trifluorométhyl) but-1-èn-3-ynyl) 2,2-diméthyl cyclopropane carboxylate de (2,6-difluorophényl)] méthyle.

### EXEMPLE 55 : [1R-[1alpha, 3alpha(E)]] 3-(2-trifluorométhyl) pent-1-èn-3-ynyl) 2,2-diméthyl cyclopropane carboxylate de (2,3-difluorophényl)] méthyle.

Rf = 0,2 (Hexane-CH₂Cl₂ 7-3).

### PREPARATION 9 : Acide [1R-[1alpha, 3alpha(E)]] 3-(2-trifluorométhyl) pent-1-èn-3-ynyl) 2,2-diméthyl cyclopropane carboxylique.

On opère comme à l'exemple 1 au départ de l'acide [1R-[(1alpha, 3alpha (E)]] 3-(2-bromo 2-(trifluorométhyl)] éthényl cyclopropane carboxylique obtenu selon EP 010874 ou BF 2 392 964 et du 1-(triméthylsilyl) propyne et obtient le produit attendu.

### EXEMPLE 56 : Préparation d'un concentré soluble

On effectue un mélange homogène de :
- Produit de l'exemple 1: : 0,25 g
- Butoxyde pipéronyle: : 1,00 g
- Tween 80: : 0,25 g
- Topanol A: : 0,1 g
- Eau: : 98,4 g

### EXEMPLE 57 : Préparation d'un concentré émulsifiable

On mélange intimement :
- Produit de l'exemple 2: : 0,015 g
- Butoxyde de pipéronyle: : 0,5 g
- Topanol A: : 0,1 g
- Tween 80: : 3,5 g
- Xylène: : 95,885 g

### EXEMPLE 58 : Préparation d'un concentré émulsifiable

On effectue un mélange homogène de :
- Produit de l'exemple 3: : 1,5 g
- Tween 80: : 20,00 g
- Topanol A: : 0,1 g
- Xylène: : 78,4 g

### EXEMPLE 59 : Préparation de granulés

On a préparé des granulés contenant de 0,1 % à 5 % de substances actives.

### ETUDE BIOLOGIQUE

### A - Activité sur Diabrotica

Les insectes tests sont des larves de dernier stade de Diabrotica.

On traite une rondelle de papier filtre de 9 cm de diamètre, déposée au fond d'une boîte de Pétri, à l'aide de 2 cm³ de solution acétonique du produit à tester. Après séchage on dépose 10 larves par dose et on effectue le contrôle de mortalité 24 heures après le traitement.

Dès la dose de 10 ppm la plupart des produits de l'invention et notamment ceux des exemples 33, 35, 43 et 45 présentent une bonne activité.

### B - Etude de l'effet d'abattage sur mouche domestique

Les insectes tests sont des mouches domestiques femelles âgées de 4 jours. On opère par pulvérisation en chambre de Kearns et March en utilisant comme solvant un mélange d'acétone (5 %) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Les produits de l'invention présentent une bonne activité.

### C - Etude de l'effet létal sur larves de Spodoptera Littoralis

Les essais sont effectués par application topique d'une solution acétonique du produit à tester, à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24°C et 65 % d'humidité relative. Après traitement les individus sont placés sur milieu nutritif artificiel (milieu de Poitout).

On effectue le contrôle des mortalités 48 heures après traitement.

Dès la dose de 5 mg/l les produits de l'exemple 1 et 5 présentent une bonne activité.

### D - Etude acaricide des composés de l'invention

On utilise des plants de haricot comportant 2 feuilles, infestées de 25 femelles de Tetranychus Urticae par feuille et mis sous bonette aérée sous plafond lumineux en lumière constante. Les plants sont traités au pistoler Fischer : 4 ml de solution toxique par plant d'un mélange à volume égal d'eau et d'acétone. On laisse sécher pendant 12 heures puis on procède à l'infestation. Les contrôles de mortalité sont effectués 80 heures après. La dose utilisée est de 5 g de produit par hl. On détermine la concentration létale 50 (CL 50). Les produits de l'invention présentent une bonne activité.

### E - Etude de l'effet létal sur Aphis cracivora

On utilise des adultes après 7 jours et l'on emploie 10 Aphis par concentration utilisée. On utilise une méthode de contact-ingestion. On effectue le traitement au pistolet de Fisher d'une feuille de fève que l'on dépose dans une boîte de Pétri en matière plastique sur une rondelle de papier humidifiée. Le traitement est effectué à l'aide de 2 ml de solution acétonique de produit à tester (1 ml par face de feuille). L'infestation par insecte est effectuée après séchage de la feuille. On maintient les insectes en contact avec la feuille pendant 1 heure. On place les insectes sur des feuilles non traitées et contrôle la mortalité au bout de 24 heures.

Les produits de l'invention présentent une bonne activité et notamment les produits des exemples 1, 47 et 53.

### F) Etude de l'effet d'abattage sur blattes

Le test est effectué sur blattes mâles (Blattella germanica), âgées de 12 semaines. On opère par pulvérisation directe dans une enceinte en verre de 13,5 cm de diamètre.

Le produit est mis en solution dans l'Isopar. On pulvérise 0,75 ml de solution pendant 2,5 secondes sur 20 blattes ayant été préalablement déposées dans l'enceinte.

On détermine le pourcentage de blattes KD au bout de 5 mn à la dose de 100 mg/l.

Les produits de l'invention présentent une bonne activité, notamment les produits des exemples 22, 32, 34, 48, 49, 50, 51 et 52.

## Revendications

**1)** Les composés de formule (I) : 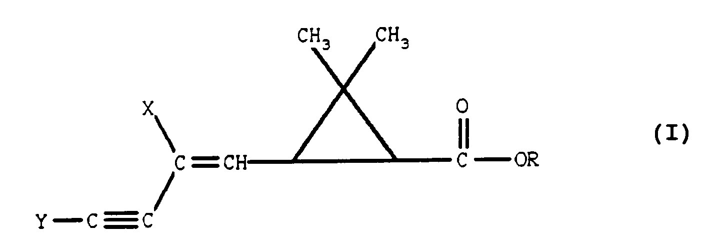 sous toutes leurs formes stéréoisomères possibles ainsi que leurs mélanges dans lesquels :
- X représente un atome d'hydrogène, un atome d'halogène, un radical alkyle renfermant jusqu'à 8 atomes de carbone, linéaire, ramifié ou cyclique, saturé ou insaturé, éventuellement substitué par un ou plusieurs atomes d'halogène,
- Y représente un atome d'hydrogène, un atome d'halogène, un radical alkyle linéaire, ramifié ou cyclique, saturé ou insaturé, renfermant jusqu'à 8 atomes de carbone, éventuellement substitué par un ou plusieurs atomes d'halogène, par un ou plusieurs radicaux CN, par un ou plusieurs radicaux hydroxyle ou alkoxy, ou Y représente un radical aryle ou aralkyle renfermant jusqu'à 16 atomes de carbone éventuellement substitué par un ou plusieurs atomes d'halogène, par un ou plusieurs radicaux alkyle ou alkoxy renfermant jusqu'à 8 atomes de carbone, ou Y représente un radical (CH₂)ₘ Si(alc₁)₃, un radical (CH₂)ₙ Oalc₂ ou (CH₂)ₚ Salc₃, m, n et p représentant un nombre entier pouvant varier de 0 à 6, alc₁, alc₂ et alc₃ représentant un radical alkyle linéaire, ramifié ou cyclique renfermant jusqu'à 8 atomes de carbone et R représente un radical alkyle renfermant jusqu'à 18 atomes de carbone ou le reste d'un alcool utilisé en série pyréthrinoïde.

**2)** Les composés de formule (I) tels que définis à la revendication 1, dans lesquels X représente un atome d'hydrogène ou de fluor ou un radical trifluorométhyle.

**3)** Les composés de formule (I) tels que définis à la revendication 1 ou 2, dans lesquels Y représente un atome d'hydrogène.

**4)** Les composés de formule (I) tels que définis à l'une quelconque des revendications 1 ou 2 dans lesquels Y représente un radical alkyle renfermant jusqu'à 6 atomes de carbone.

**5)** Les composés de formule (I) tels que définis à la revendication 4, dans lesquels Y représente un radical méthyle, isopropyle ou tert-butyle.

**6)** Les composés de formule (I) tels que définis à l'une quelconque des revendications 1 ou 2, dans lesquels Y représente un radical Si(alc₁)₃ dans lequel alc₁ conserve sa signification précédente.

**7)** Les composés de formule (I) tels que définis à la revendication 6, dans lesquels alc₁ représente un radical méthyle.

**8)** Les composés de formule (I) tels que définis à l'une quelconque des revendications 1 à 7, dans lesquels la géométrie de la double liaison est cis.

**9)** Les composés de formule (I) tels que définis à l'une quelconque des revendications 1 à 8, dans lesquels R représente :
- soit un radical alkyle renfermant de 1 à 18 atomes de carbone,
- soit un radical benzyle éventuellement substitué par un ou plusieurs radicaux choisis dans le groupe constitué par :
. les atomes d'halogène et notamment le fluor,
. les radicaux alkyle, alkényle ou alkynyle, O-alkyle, O-alkényle ou O-alkynyle, S-alkyle, S-alkényle ou S-alkynyle, linéaires, ramifiés ou cycliques renfermant jusqu'à 8 atomes de carbone éventuellement substitués par un ou plusieurs atomes d'halogène et notamment par un ou plusieurs atomes de fluor,
. les radicaux NO₂, CN, NH₂,
- soit un radical : 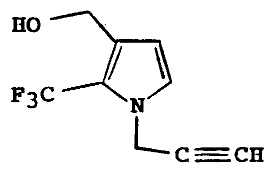
- soit un groupement : 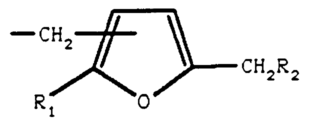 dans lequel le substituant R₁ représente un atome d'hydrogène ou un radical méthyle et le substituant R² un aryle monocyclique ou un groupement -CH₂-C≡CH et notamment un groupement (5-benzyl 3-furyl) méthyle,
- soit un groupement : 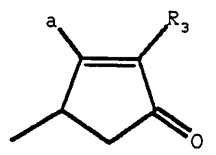 dans lequel a représente un atome d'hydrogène ou un radical méthyle et R₃ représente un radical organique aliphatique comportant de 2 à 6 atomes de carbones et une ou plusieurs insaturations carbone-carbone et notamment le radical -CH₂-CH=CH₂, -CH₂-CH=CH-CH₃, -CH₂-CH=CH-CH=CH₂, -CH₂-CH=CH-CH₂-CH₃,
- soit un groupement : 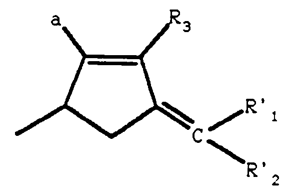 dans lequel a représente un atome d'hydrogène ou un radical méthyle, R₃ conserve la même signification que précédemment, R'₁ et R'₂, identiques ou différents, représentent un atome d'hydrogène, un atome d'halogène, un radical alkyle renfermant de 1 à 6 atomes de carbone, un radical aryle comportant de 6 à 10 atomes de carbone, un groupement alkyloxycarbonyle comportant de 2 à 5 atomes de carbone, ou un groupement cyano,
- soit un groupement : 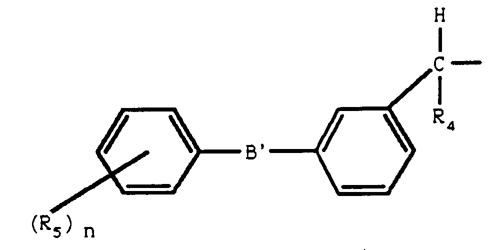 dans lequel B' représente un atome d'oxygène ou de soufre ou un groupement 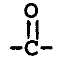 ou -CH₂- ou un groupement sulfoxyde ou un groupement sulfone et R₄ représente un atome d'hydrogène, un radical -C≡N, un radical méthyle, un radical -CONH₂, un radical -CSNH₂ ou un radical -C≡CH, R₅ représente un atome d'halogène ou un radical méthyle et n représente un nombre égal à 0, 1 ou 2, et notamment le groupement 3-phénoxy benzyle, alpha-cyano 3-phénoxy benzyle, alpha-éthynyl 3-phénoxy benzyle, 3-benzoyl benzyle, 1-(3-phénoxy phényl) éthyle ou alpha-thioamido 3-phénoxy benzyle,
- soit un groupement : 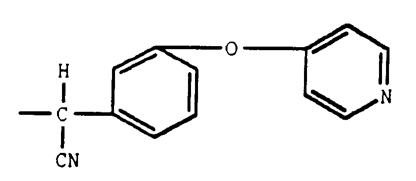
- soit un groupement : 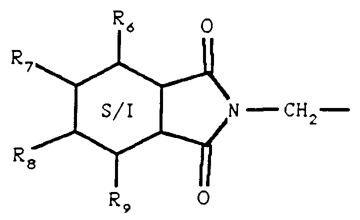 dans lequel les substituants R₆, R₇, R₈, R₉ représentent un atome d'hydrogène, un atome de chlore, ou un radical méthyle et dans lequel S/I symbolise un cycle aromatique ou un cycle analogue dihydro ou tétrahydro,
- soit un groupement : 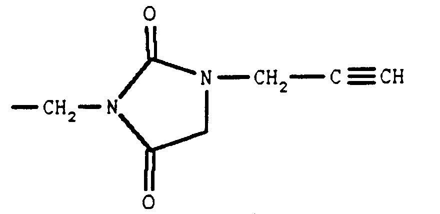
- soit un groupement : 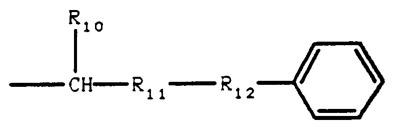 dans lequel R₁₀ représente un atome d'hydrogène ou un radical CN, R₁₂ représente un radical -CH₂- ou un atome d'oxygène, R₁₁ représente un radical thiazolyle ou thiadiazolyle dont la liaison avec 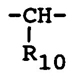 peut se trouver à l'une quelconque des positions disponibles, R₁₂ étant lié à R₁₁ par l'atome de carbone compris entre l'atome de soufre et un atome d'azote,
- soit un groupement : 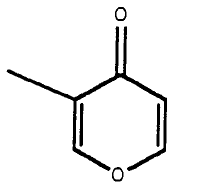
- soit un groupement : 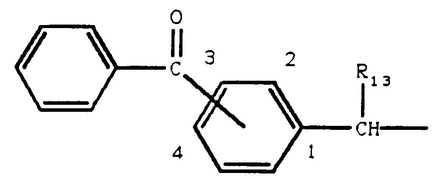 dans lequel R₁₃ représente un atome d'hydrogène, un radical méthyle, éhynyle ou cyano, et le radical benzoyle est en position 3 ou 4,
- soit un groupement : 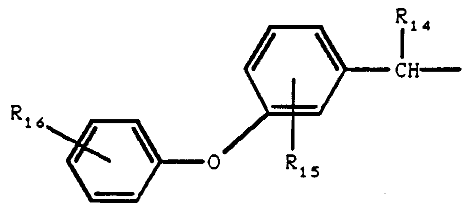 dans lequel R₁₄ a la signification indiquée ci-dessus pour R₁₃ et, R₁₅ et R₁₆, différents, représentent un atome d'hydrogène, de fluor ou de brome,
- soit un groupement : 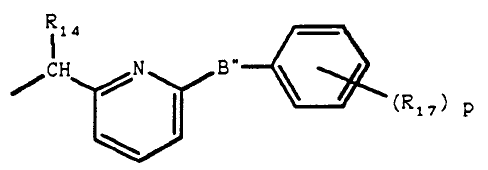 dans lequel R₁₄ est défini comme ci-dessus, chacun des R₁₇ représente indépendamment un groupement alkyle renfermant de 1 à 4 atomes de carbone, alkoxy renfermant de 1 à 4 atomes de carbone, alkylthio renfermant de 1 à 4 atomes de carbone, alkylsulfonyle renfermant de 1 à 4 atomes de carbone, trifluorométhyle, 3,4-méthylène dioxy, chloro, fluoro ou bromo, p représente un nombre égal à 0, 1 ou 2 et B" représente un atome d'oxygène ou un atome de soufre,
- soit un groupement 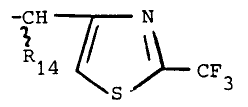
dans lequel R₁₄ a la signification indiquée précédemment.

**10)** Les composés de formule (I) tels que définis à la revendication 9 dans lesquels R représente un radical : 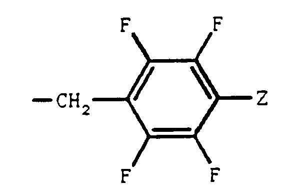 dans lequel Z représente un atome d'hydrogène, un atome de fluor, un radical NH₂, un radical alkyle, alkényle ou alkynyle O-alkyle, O-alkényle, S-alkyle ou S-alkényle, linéaires ramifiés ou cycliques renfermant jusqu'à 8 atomes de carbone, éventuellement substitués par un ou plusieurs atomes d'halogène, et notamment par un ou plusieurs atomes de fluor.

**11)** Les composés de formule (I) définis à la revendication 10 dans lesquels Z représente :
H, F, CH₃, CF₃, OCF₃, OCHF₂, CH₂C≡CH et CH₂-CH=CH₂.

**12)** Les composés de formule (I) tels que définis à la revendication 1, dans lesquels R représente le radical :

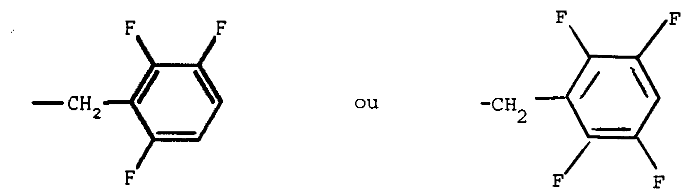

**13)** Les composés de formule (I) tels que définis à la revendication 1 dont les noms suivent :
- [1R-[1alpha, 3alpha(Z)]] 3-(2-fluoro 5,5-diméthyl hex-1-èn-3-ynyl) 2,2-diméthyl cyclopropanecarboxylate de (2,3,5,6-tétrafluoro 4-méthyl phényl) méthyle
- [1R-[1alpha, 3alpha(Z)]] 2,2-diméthyl 3-(4-(triméthylsilyl) but-1-èn-3-ynyl] cyclopropanecarboxylate de (2,3,5,6-tétrafluoro 4-(2-propynyl) phényl] méthyle
- [1R-[1alpha, 3alpha(Z)]] 3-(but-1-èn-3-ynyl) 2,2-diméthyl cyclopropanecarboxylate de (2,3,5,6-tétrafluoro 4-(2-propynyl) phényl] méthyle
- [1R-(1alpha, 3alpha(E)]] 3-(2-fluoro but-1-èn-3-ynyl) 2,2-diméthyl cyclopropanecarboxylate de (pentafluorophényl) méthyle
- [1R-[1alpha, 3alpha(E)]] 3-(2-fluoro but-1-èn-3-ynyl) 2,2-diméthyl cyclopropanecarboxylate de (2,3,5,6-tétrafluoro 4-méthyl phényl) méthyle
- [1R-[1alpha, 3alpha(E)]] 3-(2-fluoro 5,5-diméthyl hex-1-èn-3-ynyl) 2,2-diméthyl cyclopropanecarboxylate de (2,3,5,6-tétrafluoro phényl) méthyle
- [1R-[1alpha, 3alpha(E)]] 3-(2-fluoro 5,5-diméthyl hex-1-èn-3-ynyl) 2,2-diméthyl cyclopropanecarboxylate de (pentafluorophényl) méthyle
- [1R-[1alpha, 3alpha(E)]] 3-(2-fluoro 5-méthyl hex-1-èn-3-ynyl) 2,2-diméthyl cyclopropanecarboxylate de (2,3,5,6-tétrafluoro 4-méthyl phényl) méthyle.

**14)** Les composés définis à la revendication 1 dont les noms suivent :
le [1R-[1alpha, 3alpha(E)]] 3-(2-fluoro but-1-èn-3-ynyl) 2,2-diméthyl cyclopropanecarboxylate de (2,3,6-trifluoro phényl) méthyle,
le [1R-[1alpha, 3alpha(E)]] 3-(2-fluoro but-1-èn-3-ynyl) 2,2-diméthyl cyclopropanecarboxylate de (2,3,5,6-tétrafluoro phényl) méthyle,
le [1R-[1alpha, 3alpha(E)]] 3-(2-trifluorométhyl) but-1-èn-3-ynyl) 2,2-diméthyl cyclopropane carboxylate de (2,3,6-trifluorophényl) méthyle,
le [1R-[1alpha, 3alpha(E)]] 3-(2-trifluorométhyl) but-1-èn-3-ynyl) 2,2-diméthyl cyclopropane carboxylate de (2,3,5,6-tétrafluorophényl) méthyle.

**15)** Les composés définis à la revendication 1 dont les noms suivent :
le [1R-[1alpha, 3alpha(E)]] 3-(2-trifluorométhyl) but-1-èn-3-ynyl) 2,2-diméthyl cyclopropane carboxylate de (2,6-difluorophényl)] méthyle,
le [1R-[1alpha, 3alpha(E)]] 3-(2-trifluorométhyl) pent-1-èn-3-ynyl) 2,2-diméthyl cyclopropane carboxylate de (2,3-difluorophényl)] méthyle.

**16)** Procédé de préparation des composés de formule (I) tels que définis à l'une quelconque des revendications 1 à 12, caractérisé en ce que l'on soumet un acide de formule (II) : 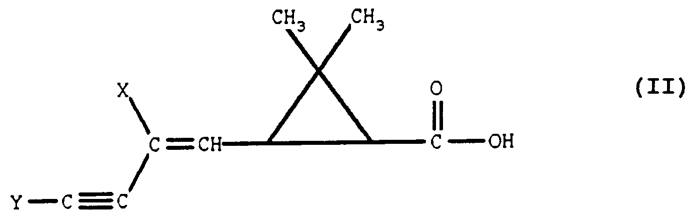 dans laquelle X et Y conservent leur signification précédente, ou un dérivé fonctionnel de cet acide à l'action d'un alcool de formule (III) :
ROH (III)
dans laquelle R conserve sa signification précédente ou d'un dérivé fonctionnel de cet alcool pour obtenir le composé de formule (I) correspondant.

**17)** A titre de produits chimiques nouveaux, les composés de formule (II) tels que définis à la revendication 16.

**18)** Variante du procédé selon la revendication 16, caractérisé en ce que l'on soumet un composé de formule (IV) : 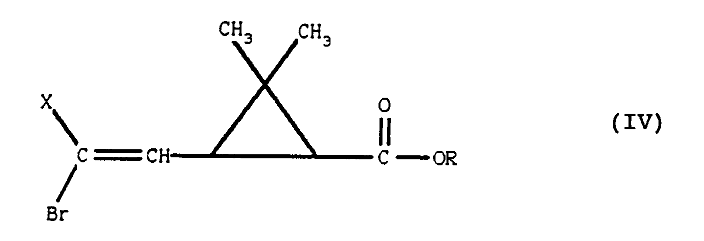 à l'action d'un agent susceptible de remplacer l'atome de brome par le radical C≡CY pour obtenir le composé de formule (I) correspondant.

**19)** Application des composés de formule (I) tels que définis à l'une quelconque des revendications 1 à 15, à la lutte contre les parasites des végétaux et les parasites des locaux.

**20)** Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 15.

**21)** Les compositions insecticides, caractérisées en ce qu'elles renferment comme principe actif au moins un composé défini à l'une quelconque des revendications 1 à 15.

**22)** Les compositions insecticides définies à la revendication 21, caractérisées en ce qu'elles sont destinées à la lutte contre DIABROTICA et les autres parasites du sol.

**23)** Les compositions acaricides, caractérisées en ce qu'elles renferment comme principe actif au moins un composé défini à l'une quelconque des revendications 1 à 15.

**24)** Associations douées d'activité insecticide, acaricide ou nématicide, caractérisées en ce qu'elles contiennent comme matière active, d'une part, un au moins des composés de formule générale (I) et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools alphacyano 3-phénoxy benzylique des acides 2,2-diméthyl 3-(2,2-dichlorovinyl) cyclopropane-1-carboxyliques, par les esters d'alcool alpha cyano 3-phénoxy benzylique d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alphacyano 3-phénoxy benzylique des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés de formule (I) peuvent exister sous toutes leurs formes stéréoisomères possibles de même que les copules acides et alcools des esters pyréthrinoïdes ci-dessus.
